# EUROPÄISCHE PATENTSCHRIFT

(11) **EP 0 476 111 B1**
(45) Veröffentlichungstag und Bekanntmachung des Hinweises auf die Patenterteilung: **22.06.1994**
(21) Anmeldenummer: 91907437.7
(22) Anmeldetag: 10.04.1991
(51) Int. Cl.: A61F 5/41

(54) **MODIFIZIERTER PRESSRING**
MODIFIED ERECTION RING
BAGUE MODIFIEE D'ERECTION

(30) Priorität: 10.04.1990 DE 4011536
(43) Veröffentlichungstag der Anmeldung: 25.03.1992
(73) Patentinhaber: CRIVELLARO, Jürgen, D-22145 Stapelfeld (DE)
(72) Erfinder: CRIVELLARO, Jürgen, D-22145 Stapelfeld (DE)
(74) Vertreter: Siewers, Gescha, Dr.
(86) Internationale Anmeldenummer: EP9100680
(87) Internationale Veröffentlichungsnummer: WO9115173

(56) Entgegenhaltungen:
- GB-A- 884 357
- US-A- 4 785 802

## Beschreibung

Die Erfindung betrifft einen modifizierten Pressring.

Ein elastischer Penisring, welcher in Kombination mit einem Drahtgestell, das einen Rektalstab aufweist, verwendet wird, ist aus der US-A-4 785 802 als dem nächestliegenden Stand der Technik bekannt.

Penisringe, auch als Pressringe bezeichnet, sind in vielen Kulturen seit Jahrhunderten in Verwendung und werden häufig eingesetzt, um die Potenzschwäche des Mannes zu beheben. Penisringe können aus elastischem oder nicht elastischem Material wie beispielsweise Gummi oder, wie häufig im japanischen Kulturkreis, aus Porzellan oder Elfenbein bestehen und bewirken durch Reduktion des Blutrückflusses in den Adern die Entstehung oder den Erhalt einer Erektion.

Potenzschwierigkeiten beim Mann sind nach Erkenntnissen der Sexualmediziner weit verbreitet und scheinen ständig, insbesondere im höheren Alter zuzunehmen und führen sehr oft zu psychischen und sozialen Problemen. Ein Grund für die Zunahme scheint beispielsweise auch in der verbreiteten Gabe von Betablockern gegen Herz- und Blutdruckbeschwerden bei Männern im Alter von etwa ab 40 zu liegen. Die Verwendung von Pressringen wird daher auch von Sexualmedizinern bei Potenzschwäche empfohlen.

Es hat sich aber herausgestellt, daß die Verwendung solcher Pressringe zur Stützung einer schwachen Erektion und zur Verlängerung einer Erektion häufig nicht ausreichen. Andererseits ist bekannt, daß der Analbereich und der Mastdarm sensorisch empfindlich sind und daß eine Stimulation dieses Bereiches bei vielen Männern zu einer spontanen und langdauernden Erektion führen kann.

Erfindungsgemäß wird daher ein Pressring vorgeschlagen, der dadurch gekennzeichnet ist, daß er eine einstückige Kombination mit einem Rektalstab bildet. Dadurch ist gleichzeitig eine Stimulation der sensorischen Nerven im Analbereich und im Rectum sowie die übliche pressorische Wirkung des Penisringes möglich. Bei psychischer bedingter Potenzschwäche lassen sich durch diese Kombination beträchtliche Erfolge erzielen, die zu einer spontanen und anhaltenden Erektion führen.

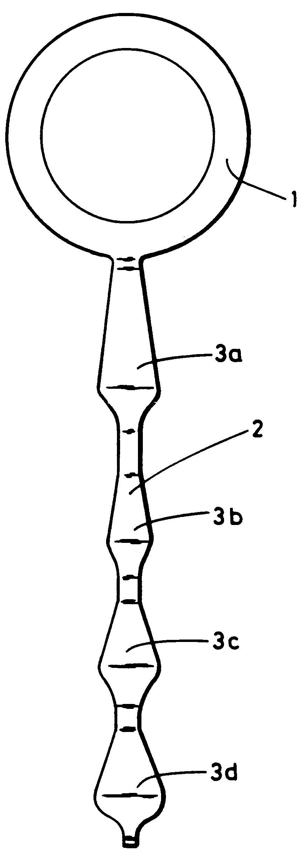

Die Erfindung wird im folgenden anhand der Zeichnung näher erläutert:

Die Figur zeigt einen erfindungsgemäßen Pressring in Draufsicht. Der Pressring 1 ist einstückig mit einem Rektalstab 2 verbunden, der vorzugsweise Verdickungen 3a, 3b, 3c, 3d unterschiedlichen Durchmessers zur Verstärkung der Stimulation aufweist. Der Pressring besteht aus physiologisch unbedenklichen Kunststoffen, und zwar insbesondere aus modifizierten Styrol-Butadien-Styrol-Blockcopolymeren, die sich durch große Flexibilität, hohes Rückstellvermögen und starke Haftreibung auszeichnen. Die verwendeten Kunststoffe sollten eine Shorehärte im Bereich zwischen etwa 30 - 60 und vorzugsweise um 40 aufweisen. Die vorzugsweise eingesetzten Styrol-Butadien-Styrol-Blockcopolymeren lassen sich bei allen thermoplastischen Arbeitsverfahren leicht verarbeiten und zeichnen sich insbesondere dadurch aus, daß eine Formung ohne Gratbildung möglich ist.

## Patentansprüche

1. Pressring, gekennzeichnet durch eine einstückige Kombination mit einem Rektalstab (2).

2. Pressring nach Anspruch 1, gekennzeichnet durch Verdickungen (3a, 3b, 3c, 3d) des Rektalstabes (2) mit unterschiedlichen Durchmessern.

3. Pressring nach Anspruch 1 oder 2, dadurch gekennzeichnet, daß er aus physiologisch unbedenklichen Kunststoffen besteht.

4. Pressring nach Anspruch 1 bis 3, dadurch gekennzeichnet, daß der Kunststoff eine Shorehärte zwischen etwa 30 - 60, vorzugsweise um 40 aufweist.

5. Pressring nach Anspruch 1 bis 4, dadurch gekennzeichnet, daß er aus modifizierten Styrol-Butadien-Styrol-Blockcopolymeren besteht.

## Claims

1. Erection ring characterized by a one-piece combination with a rectal rod (2).

2. Erection ring according to claim 1, characterized by enlargements (3a, 3b, 3c, 3d) of different diameters of the rectal rod (2).

3. Erection ring according to claim lor 2, characterized in that it consists of physiologically harmless plastic material.

4. Erection ring according to claims 1 to 3, characterized in that the plastic material has a Shore hardness between approximately 30 - 60, preferably around 40.

5. Erection ring according to claimsl to 4, characterized in that it consists of modified styrene-butadiene-styrene block copolymers.

## Revendications

1. Bague d'érection caractérisé par une combinaison avec un bâton rectal (2) tout d'un tenant.

2. Bague d'érection selon revendication 1, caractérisé par des gonflements (3a, 3b, 3c, 3d) du bâton rectal de plusieurs diamètres.

3. Bague d'érection selon les revendications 1 ou 2, caractérisé en ce qu'il se compose de matière plastique qui n'offre physiologiquement aucun inconvénient.

4. Bague d'érection selon les revendications 1 à 3, caractérisé en ce que la matière plastique montre une dureté Shore entre à peu près 30 - 60, de préférence autour de 40.

5. Bague d'érection selon les revendications 1 à 4, caractérisé en ce qu'il se compose de bloc-copolymères modifiées de styrène-butadiène-styrène.
